# EUROPEAN PATENT SPECIFICATION

(11) **EP 2 506 721 B1**
(45) Date of publication and mention of the grant of the patent: **14.09.2016**
(21) Application number: 10803171.7
(22) Date of filing: 02.12.2010
(51) Int. Cl.: A23K 50/00, A23K 20/00

(54) **METHODS AND FEED COMPOSITIONS FOR MASKING OF FISH SEMIOCHEMICALS**
METHODEN UND TIERFUTTERMITTELZUBEREITUNGEN ZUR MASKIERUNG DER SEMIOCHEMIKALIEN VOM FISCH
METHODES ET COMPOSITIONS D'ALIMENTS POUR ANIMAUX POUVANT MASQUER LES AGENTS SEMIOCHIMIQUES DES POISSONS

(30) Priority: 02.12.2009 NO 20093460
(43) Date of publication of application: 10.10.2012
(60) Divisional application: 12172507.1
(73) Proprietor: Ewos Innovation AS, 4335 Dirdal (NO)
(72) Inventor: WADSWORTH, Simon, 9405 Harstad (NO); VECINO, Dr. José Luis González, 4326 Sandnes (NO); PINO, Jorge, Puerto Varas (CL); MORDUE, Jenny, AB15 9AQ Aberdeen (GB)
(74) Representative: Acapo AS
(86) International application number: PCT/NO2010/000442
(87) International publication number: WO 2011/068415

(56) References cited:
- EP-A1- 1 208 751
- WO-A1-00/59316
- WO-A1-2008/049437
- WO-A2-2004/084645
- WO-A2-2004/091307
- DE-U1- 20 010 027
- GB-A- 2 210 616
- GB-A- 2 388 544
- KR-A- 20090 093 518
- US-A- 3 662 069
- US-A- 5 504 081
- US-A1- 2002 164 384
- US-A1- 2005 266 052
- US-A1- 2008 026 083
- ROTH M ET AL: "Current practices in the chemotherapeutic control of sea lice infestations in aquaculture: A review", JOURNAL OF FISH DISEASES, OXFORD, GB, vol. 16, no. 1, 1 January 1993 (1993-01-01), pages 1-26, XP009146063, ISSN: 0140-7775
- DATABASE WPI Week 199039 Thomson Scientific, London, GB; AN 1990-294657 XP002638541, & JP 2 207758 A (TAIYO KAGAKU KK) 17 August 1990 (1990-08-17)
- SYLVESTRE M., LEGAULT J., DUFOUR D., PICHETTE A.: "Chemical composition and anticancer activity of leaf essential oil of Myrica gale L.", PHYTOMEDICINE, vol. 12, no. 4, 2004, pages 299-304, XP002638542, DOI: 10.1016/j.phymed.2003.12.004

## Description

### FIELD OF THE INVENTION

The present invention relates to a composition, compound and feed composition for use in masking the odor of fish semiochemicals in water wherein the attraction between an parasite and a fish is reduced.

### BACKGROUND TO THE INVENTION

Sea lice *(Lepeophtheirus salmonis, Caligus* sp*.)* are the major pathogen currently affecting the global salmon farming industry and have a significant impact on many areas of production. Economic impact on the aquaculture industry is high due to high annual losses. There is also continued concern over the impact of salmon farming on wild salmon populations with increased density of sea lice adjacent to these production sites. Control measures have been reliant upon the use of a number of chemotherapeutants since the 1970's. Reduced efficacy has now been reported for all compounds, with the exception of the insect growth regulators (IGR) diflubenzuron and teflubenzuron. Further methods are therefore required to effectively control sea lice, in conjunction with sea lice medicines.

The publication by Roth M et al., "Current practices in the chemotherapeutic contro of sea lice infestations in aquaculture: A review", Journal of Fish Diseases, vol. 16, no. 1, 1 January 1993, pages 1-26, XP009146063 discloses the use of garlic-coated salmon pellets in an attempt to reduce lice numbers in salmon.

The publications Database WPI, Week 1993039, Thomson Scientific, London, GB; AN 1990-294657, XP002638541 &JP 2 207758 A (Taiyo Kagaku KK), 17. August 1990 discloses the use of garlic to prevent bacterial infections in fish.

### Host-specific parasites

The *Lepeophtheirus* genus of sea lice is a host-specific parasite. *L. salmonis* will only complete its life cycle on salmonid species, although mobile stages may occasionally be observed as opportunists on additional fish types. Other *Lepeophtheirus* sp. will target a narrow range of other fish species.

### Immune suppression of the host

The *Lepeophtheirus* genus of sea lice has evolved a range of mechanisms to suppress the immune response of their particular hosts. To overcome a potentially fatal inflammatory reaction the sea lice release a series of secretary / excretory products (SEP) into the host tissue, via salivary glands. Prostaglandins (PGE₂), alkaline phosphatase and a range of trypsin-like proteases have been identified as sea lice SEPs. It is thought that several additional unidentified factors such as phosphatase, apyrase and macrophage inhibition factor are also present.

### Effect of immune suppressants

*L. salmonis* has a significant immunosuppressive effect on a range of responses in Atlantic salmon including reduced respiratory burst, lower macrophage activity, increased apoptosis, necrosis, decreased numbers of mucosal cells and down-regulation of immune genes such as interleukin IL-1ß and MHC-1. Suppression occurs at localised attachment sites, although a more generalised effect may occur with higher levels of sea lice infection. Once they have suppressed the immune system of the host, the lice are able to extend a frontal filament for a secure attachment. This is intimately associated with the host tissues and able to survive any subsequent immune response from that species.

### A fatal risk of attaching to the wrong host

*Lepeophtheirus* sp. are not able to suppress the immune system of non-host species. If lice try and settle on to a resistance fish species the immune response will kill it. Thus correct identification of the host is essential for attachment and survival of *Lepeophtheirus* sp.

### Correct host identification

Sea lice have advanced olfactory and contact chemoreceptors that are capable of accurate identification of specific host molecules. Semiochemicals (behaviour-modifying chemicals) are used by a range of arthropods in chemical communication systems to locate a host, mate or oviposition site. Similarly, many copepods use chemical cues to identify and seek out mates.

### Caligus species

Lice within the *Caligus* genus have an extensive range of potential hosts; C *elongatus* is known to infect over 80 host species world wide. *Caligus* have been found to posses a greater range and quantity of serine and non-serine proteases than *L. salmonis* and this may assist in defeating a greater range of immune responses from many different species. In addition *Caligus* deploy a different attachment mechanism that is not as intimately associated with host tissue. *Caligus* remove the epidermal tissue from the scales and then the frontal filament attaches directly to the cleared scales via a basal plate. The frontal filament is much longer than that deployed by *L. salmonis* and this allows the louse to remain at some distance from the host immune system. Despite these generalists adaption's some *Caligus* species still demonstrate a high degree of host specificity. This may develop in populations in areas where a particular host population is abundant such as *Caligus rogercresseyi* which are now the dominant sea lice species on salmon farms in Chile.

Through behavioral trials, tested the hypothesis that the inter-and intraspecific relationships of salmon louse, *C. rogercresseyi* are mediated by semiochemical compounds has been tested. It has been shown that the host species studied, Coho salmon, Atlantic salmon, and Rainbow trout, emit chemical signals that attract sea lice.

The object of the present invention is to provide a feed composition for prevention and control of sea lice attraction to, and infections in fish, preferably Salmonidae that is easily applicable, effective in long-term use and are considered as environmentally friendly and less toxic than many known chemotherapeutants. In particular, an object of the present invention is to provide a feed composition and a method for masking the semiochemical compounds in order to reduce the attraction of a sea lice for salmonidae.

Patent document GB 2 388 544 discloses the use of fish semiochemicals to attract parasites onto e.g., traps or killing or sterilising agents, or to use these in combination with e.g., masking agents.

### SUMMARY OF THE INVENTION

A first aspect of the present invention relates to a composition for use in masking the odor of a fish semiochemical in water, wherein the attraction between a parasite and a fish in water is reduced, characterized in that an extract or compound is added to said water or is administered to a fish in said water, wherein said extract or compound is selected from;
i) an extract of garlic, or
   ii) a compound of formula (I);

      **R¹-S-S-R¹** (I)
   wherein each R¹ independently of each other is C₁-C₄ alkyl or C₂-C₃ alkenyl or C₂-C₃ alkynyl.

In a preferred embodiment, said fish semiochemical is isophorone. In a preferred embodiment is said fish semiochemical 1-Octen-3-ol. In a preferred embodiment is said fish semiochemical 6-methyl-5-hepten-2-one.

Preferably is said fish is a Salmonidae.

Preferably, is said water Salmonidae conditioned sea water or said fish in the water is a Salmonidae.

Preferably, said Salmonidae is selected from the group consisting of Atlantic salmon, coho salmon, Chinook, rainbow trout, Arctic charr and other farmed salmon species.

Preferably, said parasite is an ectoparasite, more preferably sea lice (*Lepeophtheirus salmonis, Caligus* sp.*).*

In a preferred embodiment is said compound a compound of formula (I) above.

Preferably, at least one R¹ is -CH₂-CH=CH₂ or -CH=CH-CH₃. Preferable, both R¹ groups are identical, and are either -CH₂-CH=CH₂ or -CH=CH-CH₃.

Preferably, said compound is diallyl disulfide or diallyl sulphide.

A second aspect of the present invention relates to a compound for use in masking the odor of a fish semiochemical in water, wherein the attraction between an parasite and a fish is reduced, or wherein the infestation or infection of an parasite in a fish is reduced, , characterized in that characterized in that said compound is added to said water or is administered to a fish in said water, wherein said compound is a compound of formula (I);

**R¹-S-S-R¹** (I)

wherein each R¹ independently of each other is C₁-C₄ alkyl or C₂-C₃ alkenyl or C₂-C₃ alkynyl, and wherein said parasite is an ectoparasite, preferably sea lice *(Lepeophtheirus salmonis, Caligus* sp.*)*.

Preferably, said fish is a Salmonidae.

Preferably, is said water Salmonidae conditioned sea water or said fish in the water is a Salmonidae.

Preferably, said Salmonidae is selected from the group consisting of Atlantic salmon, coho salmon, Chinook, rainbow trout, Arctic charr and other farmed salmon species.

In a preferred embodiment is said compound a compound of formula (I) above.

Preferably, at least one R¹ is -CH₂-CH=CH₂ or -CH=CH-CH₃. Preferable, both R¹ groups are identical, and are either -CH₂-CH=CH₂ or -CH=CH-CH₃.

Preferably, said compound is diallyl disulfide or diallyl sulphide.

A third aspect of the present invention relates to a feed composition for use in masking the odor of a fish semiochemical in water, wherein the attraction between a parasite and a fish is reduced, or wherein the infestation or infection of a parasite in a fish is reduced, wherein the feed comprises conventional feed ingredients such as lipids, proteins, vitamins, carbohydrates and minerals, and a compound of formula (I);

**R¹-S-S-R¹** (I)

wherein each R¹ independently of each other is C₁-C₄ alkyl or C₂-C₃ alkenyl or C₂-C₃ alkynyl.

Preferably, said compound in the feed is in a concentration range of 0.01-0,5, preferably in a concentration of 0.125% by weight of the feed.

### DESCRIPTION OF THE INVENTION

Embodiments of the invention will now be described, by the way of examples with reference to the following figures:
Figure 1 a shows the directional dose response of *L. salmonis* copepodids to seawater control, 100 parts per trillion (ppt) isophorone, 100 ppt isophorone plus 100 ppt garlic oil and 100 ppt garlic oil on its own.
Figure 1b shows the activation dose response of *L. salmonis* copepodids to seawater control, 100 parts per trillion (ppt) isophorone, 100 ppt isophorone plus 100 ppt garlic oil and 100 ppt garlic oil on its own.
Figure 2a shows the directional dose response of *L. salmonis* copepodids to seawater control, salmon conditioned water (SCW), SCW plus 50 ppt diallyl disulfide and SCW plus 10 ppt diallyl sulfide.
Figure 2b shows the activation dose response of *L. salmonis* copepodids to seawater control, salmon conditioned water (SCW), SCW plus 50 ppt diallyl disulfide and 10 ppt diallyl sulfide.
Figure 3a shows the directional dose response of *L. salmonis* copepodids to seawater control, salmon conditioned water (SCW) and SCW plus 10, 1 and 0.1 ppt diallyl sulfide.
Figure 3b shows the activation dose response of *L. salmonis* copepodids to seawater control, salmon conditioned water (SCW) and SCW plus 10, 1 and 0.1 ppt diallyl sulfide.
Figure 4a shows the directional dose response of *L. salmonis* copepodids to seawater control, salmon conditioned water (SCW) and SCW plus 4-pentenyl, 2-phenylethyl and butyl isothiocyanate at 100 ppt.
Figure 4b shows the activation dose response of *L. salmonis* copepodids to seawater control, salmon conditioned water (SCW) and SCW plus 4-pentenyl, 2-phenylethyl and butyl isothiocyanate at 100 ppt.
Figure 5. Chemotaxis response of C. *rogercresseyi* Copepodid to stimulus masked with compounds B1 (A), B2(B) y B3(C) at different concentration (* P < 0,05; Chi-square test)
Figure 6. Preference Index of C. *rogercresseyi* copepodids to host signal masked with compounds B1(A), B2 (B) y B3(C) at different concentration.
Figure 7. Fish fed the butyl isothiocyanate (B1) showed a significant reduction of 42% in levels of sea lice compared to controls (Figure 2). There was a trend for a reduction in lice levels with both diallyl sulfide (B2) and diallyl disulfide (B3)

### EXPERIMENTAL SECTION

### Example 1: In vitro assessment of the effect of different compounds on Lepeophtheirus salmonis

A number of plant products were tested for their ability to mask salmon odour in order to inhibit the attractant of lice to salmon and to prevent *L. salmonis* settlement on salmon. A Y-tube behavioural arena was developed and used to test the ability of plant extracts/compounds to inhibit copepodid attraction to salmon conditioned water.

Products tested were:
- garlic constituents; garlic oil, diallyl disulphide and diallyl sulphide
- cruciferous isothiocyanates; allyl-, propyl-, butyl-, pentenyl-, phenyethyl-isothiocyanates
- plant extracts; bog myrtle, lavender, rosemary

### Material and methods:

### Lice collection

Ovigerous female *Lepeophtheirus salmonis* were collected from Atlantic salmon. Material was transported on ice to the laboratory with clean seawater for sorting. Water from the source site was collected and used for subsequent rearing of egg strings. Strings were removed gently from their point of attachment to adult females using ultra-fine forceps and placed in 2 L glass conical flasks. All flasks were aerated to keep the strings in suspension and promote hatching. Egg strings were reared under a 16 h light - 8 h dark regime and at 12°C ambient temperature in water from the source site.

Development of the egg to the copepodid was determined as a function of the mean temperature following Johnson and Albright (1991). Strings were monitored twice daily for hatching of nauplii and subsequent development to the copepodid stage, at which point they were removed for use in behavioural bioassays.

### Fish Conditioned Water

Fish conditioned water was collected as described by Devine *et al.* (2000) and Ingvarsdottir *et al.* (2002b). Atlantic salmon, S. *salar* were maintained in aquaria containing artificial seawater (32 ‰). Fish conditioned water was obtained by placing the fish for 24 h into a circulating flume (20 cm x 25 cm x 420 cm) filled with artificial seawater (100 L) circulated at a rate of 30 cm s⁻¹. Aeration was provided by bubbling compressed air into the raceway. Standardisation of fish odour in the water was achieved by using the water at a concentration of 8-10 g live fish L⁻¹ 24 h⁻¹. Conditioned water was either used immediately or frozen for later use.

### Lice Behaviour L. salmonis

A vertical Y-tube bioassay modified by Bailey *et al.* (2006) from that previously described by Ingvarsdóttir *et al.* (2002a) was used to study *L. salmonis* copepodid activation and directional (taxis) responses to host semiochemical components and potential host-masking compounds. The Y-tube was constructed from glass (1 cm diameter bore) moulded into a 'Y' design between two glass sheets of glass (2 mm thick). The arms were 6.5 cm in length and the main leg was 8 cm long. The main leg of the Y-tube was fitted with a glass stopper and filter to prevent copepodids from entering the outflow tubing running to waste. A syringe pump (SP 200 iz, World Precision Instruments, Florida, USA) held two plastic 60 mL syringes (Terumo Monoject, New Jersey, USA), which were loaded with test odours prior to use. The syringe pump was programmed to deliver a consistent flow rate of 2 mL min⁻¹. Chemical dyes demonstrated a clear demarcation of the flow down each arm and no mixing of water in the main leg of the T-tube.

When single chemical stimuli were tested e.g. salmon conditioned water (SCW), the test water was introduced to one arm whilst artificial seawater (ASW) at 32 ‰ was introduced into the other. When one of the isothiocyanates for example was tested, seawater was introduced into one arm whilst SCW plus the isothiocyanate at the desired concentration were introduced to the other. The introduction of stimuli was alternated between left and right inflow arms during each experiment, with washing in between, to eliminate positional bias. At the beginning of each experiment, the Y-tube was allowed to fill and run with seawater or seawater plus a cue/masking chemical, and a single copepodid was introduced using PTFE tubing and syringe into the tube at a point 1.5 cm above the base of the main leg. The copepodid was allowed a maximum of 3 min to respond. Each trial consisted of 1 copepodid.

Replicate tests were carried out over a period of four days to monitor for age effects of the lice on results.

Behaviour was defined by the degree of movement within the Y-Tube, as described by Ingvarsdóttir *et al.* (2002b). Behaviour was divided into two categories, low and high. Low activity was defined as the movement of the copepodid less than the length of the main leg. High activity was defined as movement of the copepodid more than the length of the main leg. Movement into either arm was also regarded as high activity. Both activation and directional responses of copepodids were measured. For directional responses, the number of copepodids choosing the stimulus arm rather than the control arm within the allocated 3 min period were compared to the control in which seawater was presented in both arms.

### Chemicals

Chemicals used in behavioural bioassays were supplied by the Chemical Ecology Group at Rothamsted Research, Harpenden, Hertfordshire, UK. Solutions of individual chemicals in ethanol (0.001, 0.01, 0.1 and 1 mg/mL) were prepared and diluted to 1 µL/L in artificial seawater (Ingvarsdóttir *et al*., 2002b) to give a final concentration of 0.1, 1, 10, 100 and 1,000 parts per trillion (ppt) respectively.

### Data Analysis

Copepodid responses to ASW (Artificial sea water) and SCW (Salmon conditioned sea water) across all experiment days were compared in the first instance using a chi-square test to determine if there was a day effect on louse behaviour. If this proved to be non-significant, it implies that the data are consistent across days and therefore can be pooled.

For directional responses and experiments on activity, the null hypothesis that all lice in all treatments behaved the same was tested using a 'global' X² contingency table (Zar, 1999). Upon rejection of that hypothesis, data were analysed by *post hoc* targeted pairwise comparisons using a 2 x 2 X² contingency table (Zar, 1999) to identify whether pairs of treatments of interest were significantly different.

Experiments testing whether allyl isothiocyanate can mask the attractiveness of salmon conditioned water were conducted in two blocks. In addition to X² analysis of the original data (block 1), binomial logistic regression was used to test whether copepodid directional and activation responses differed both between experimental treatments (salmon conditioned water presented alone, or with three concentrations of ally isothiocyanate, against an artificial seawater control) and between blocks. Two separate models were constructed, with either copepodid directional response (test or control) or activity (high or low) entered as the dependent variable. In both cases, treatment and block were entered as factors, with a treatment by block interaction included to test if louse responses to each treatment varied between blocks. Significance of terms in both models was investigated through stepwise deletion (changes in deviance assessed through X² tests) and comparisons of responses at each concentration of allyl isothiocyanate with respect to salmon conditioned water (no allyl isothiocyanate) made using Wald statistics.

### Results in vitro assessment Lepeophtheirus salmonis

### Garlic Oil

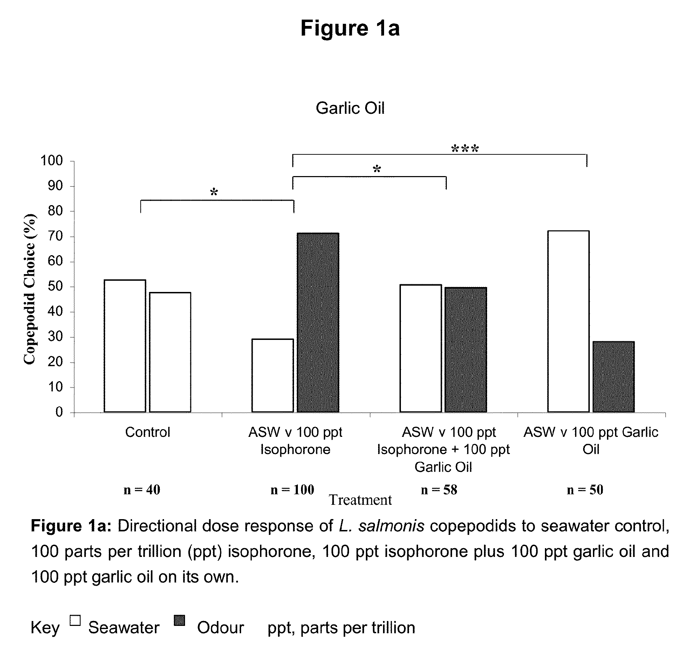

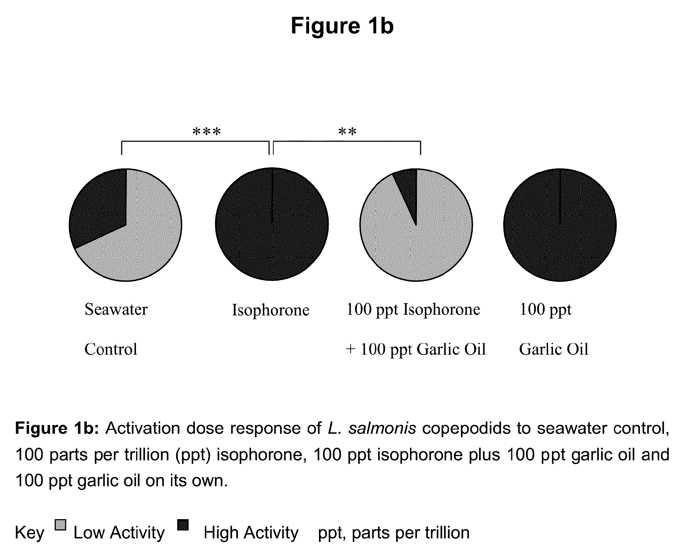

For directional responses i.e. upstream positive rheotaxis, the global X² showed that lice did not behave the same in all treatments (X² = 26.42, df = 3, P < 0.001). When compared with the seawater control, significantly more copepodids chose the arm containing the isophorone (X² = 6.87, df = 1, P < 0.01), a component of salmon conditioned water. A significant decrease in copepodid responses was detected with isophorone plus garlic oil (X² = 8.72, df = 1, P < 0.01) and with garlic oil alone (X² = 25.1, df = 1, P < 0.001) when compared against isophorone responses (Figure 1 a). The number of *L. salmonis* copepodids making directional responses, not choosing and the total number of replicates for each treatment are presented in Table 2a.

**Table 2a**

| Number of *L. salmonis* copepodids making directional responses, non-choosers and the total number of replicates for each treatment. | | | |
|---|---|---|---|
| Assay | Directional Responses | No Choice | Total No. Replicates |
| ASW Control | 40 | 36 | 76 |
| ASW v Isophorone | 100 | 0 | 100 |
| ASW v Isophorone + Garlic Oil | 58 | 21 | 79 |
| ASW v Garlic Oil | 50 | 0 | 50 |

Under control conditions, when only seawater was present in both arms of the Y tube, 68% of copepodids showed low activity, and 32% were in the high activity category. The global X² showed that lice did not behave the same in all activity treatments (X² = 72.81, df = 3, P < 0.001). When compared with the seawater control, a significant increase in high activity behaviour was observed in the presence of isophorone (X² = 36.57, df = 1, P < 0.001). Significantly more copepodids showed low activity with isophorone plus garlic oil when compared against isophorone responses (X² = 7.25, df = 1, P < 0.01) however. No difference in activity was detected between garlic oil alone and isophorone (X² = 0, df = 1, NS; Figure 1b).

### Garlic Oil Compounds: Diallyl Disulfide and Diallyl Sulphide

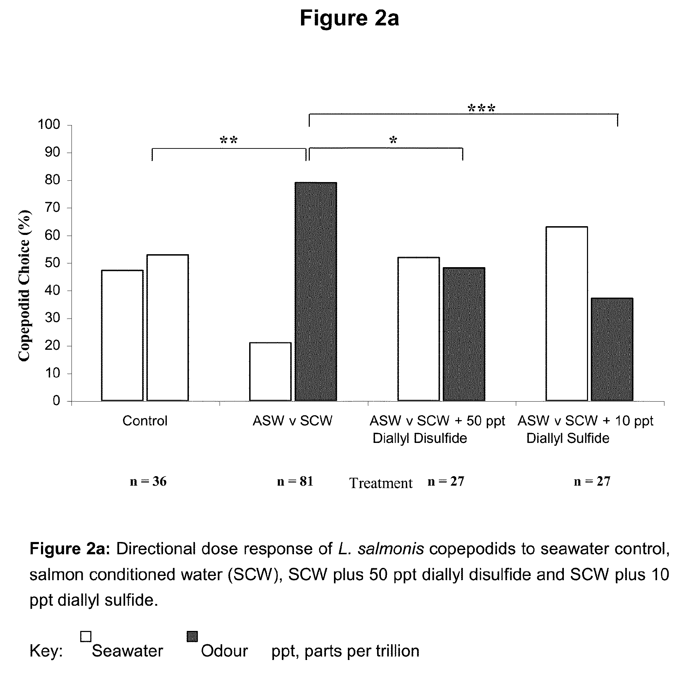

For directional responses, the global X² showed that lice did not behave the same in all treatments (X²=14.17, df = 3, P < 0.001). When compared with the seawater control, significantly more copepodids chose the arm containing the salmon conditioned water, SCW (X² = 11.82, df = 1, P < 0.001). A significant decrease in copepodid responses was seen with SCW plus 50 ppt diallyl disulfide (X² = 9.43, df = 1, P < 0.01) and SCW plus 10 ppt diallyl sulphide (X²=16.54, df = 1, P < 0.001) when compared against SCW responses (Figure 2a). The number of *L. salmonis* copepodids making directional responses, not choosing and the total number of replicates for each treatment are presented in Table 2b.

**Table 2b**

| Number of *L. salmonis* copepodids making directional responses, non-choosers and the total number of replicates for each treatment. | | | |
|---|---|---|---|
| Assay | Directional Responses | No Choice | Total No. Replicates |
| ASW Control | 36 | 84 | 120 |
| ASW v SCW | 81 | 2 | 83 |
| ASW v SCW + 50 ppt DDS | 27 | 28 | 55 |
| ASW v SCW + 10 ppt DS | 27 | 28 | 55 |

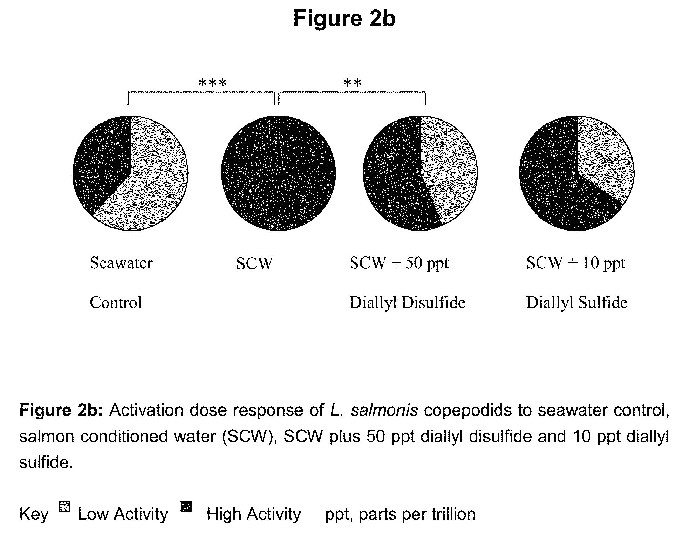

Under control conditions, when only seawater was present in both arms of the Y tube, 62% of copepodids showed low activity, and 38% were in the high activity category. The global X² showed that lice did not behave the same in all activity treatments (X²= 80.89, df = 3, P < 0.001). When compared with the seawater control, a significant increase in high activity was seen in the presence of SCW (X² = 80.54, df = 1, P < 0.001). Significantly more copepodids showed low activity in the presence of SCW plus 50 ppt diallyl disulfide (X²= 43.84, df = 1, P < 0.001) and SCW plus 10 ppt diallyl sulphide (X²= 33.25, df = 1, P < 0.001) when compared against SCW responses (Figure 2b).

### Diallyl Sulphide Dose Response

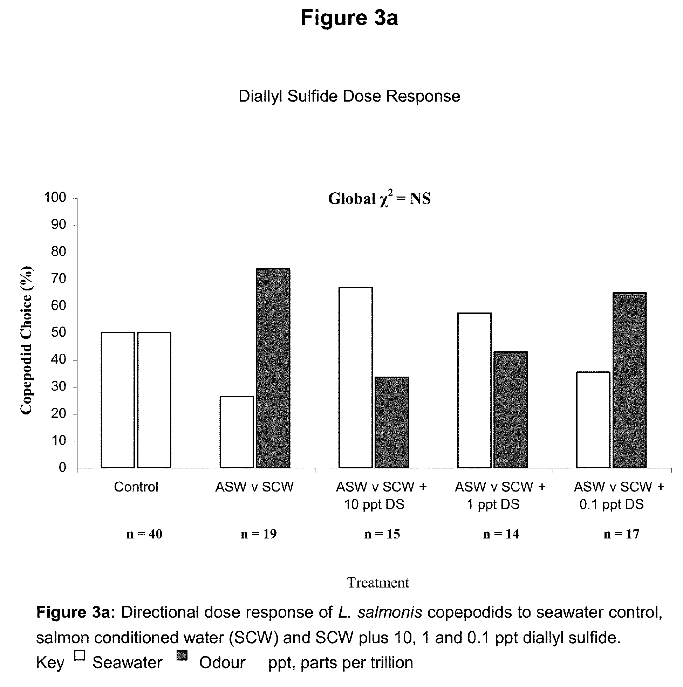

The global X² showed that lice behaved the same in all treatments (X² = 7.25, df = 4, NS) in directional response assays. As a result, further pair wise comparisons were not carried out (Figure 3a). The number of *L. salmonis* copepodids making directional responses, not choosing and the total number of replicates for each treatment are presented in Table 2c.

**Table 2c**

| Number of *L. salmonis* copepodids making directional responses, non-choosers and the total number of replicates for each treatment. | | | |
|---|---|---|---|
| Assay | Directional Responses | No Choice | Total No. Replicates |
| ASW Control | 40 | 113 | 153 |
| ASW v SCW | 19 | 1 | 20 |
| ASW v SCW + 10 ppt DS | 15 | 5 | 20 |
| ASW v SCW + 1 ppt DS | 14 | 6 | 20 |
| ASW v SCW + 0.1 ppt DS | 17 | 3 | 20 |

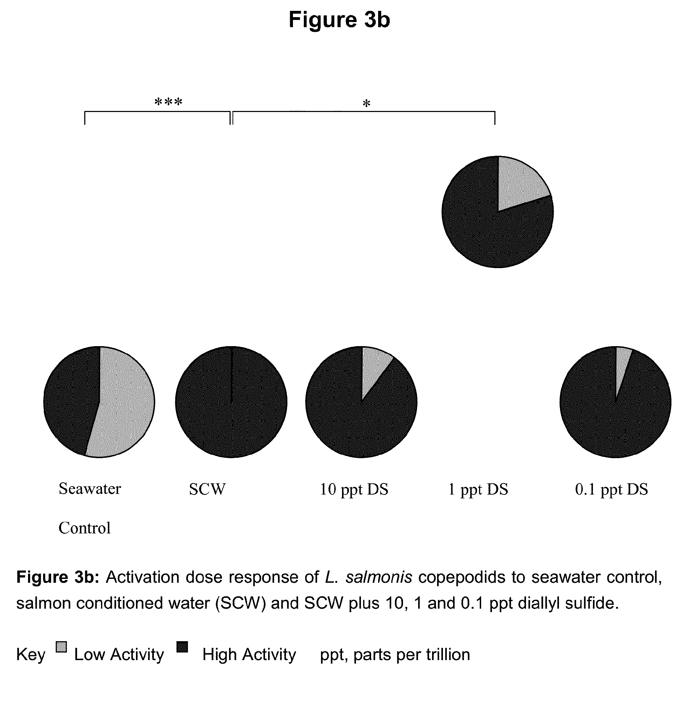

The global X² showed that lice did not behave the same in all activity treatments (X² = 42.02, df = 4, P < 0.001). When compared with the seawater control, a significant increase in high activity was detected with SCW (X² = 19.64, df = 1, P < 0.001). Significantly more copepodid showed low activity with SCW plus 1 ppt diallyl sulfide (X² = 4.44, df = 1, P < 0.05) when compared against SCW responses. No difference in activity was detected between SCW plus 0.1 (X² =1.03, df = 1, NS) and 10 ppt diallyl sulphide (X² = 2.11, df = 1, NS; Figure 3b) however.

### Isothiocyanate Compounds

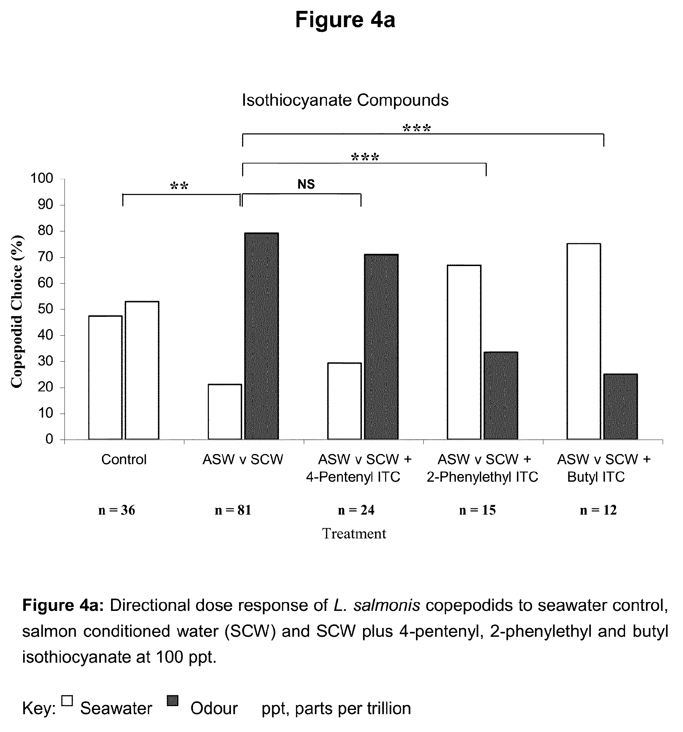

The global X² showed that lice did not behave the same in all treatments (X² = 26.50, df = 4, P < 0.001) in directional response assays. When compared with the seawater control, significantly more copepodids chose the arm containing the salmon conditioned water, SCW (X² = 11.82, df = 1, P < 0.001). A significant decrease in copepodid responses was detected with SCW plus 100 ppt 2-phenylethyl (X² = 13.06, df = 1, P < 0.001) and SCW plus 100 ppt butyl isothiocyanate (X² =15.14, df= 1, P < 0.001) when compared against SCW responses. No difference in directional responses was detected between SCW plus 100 ppt 4-pentenyl isothiocyanate and SCW responses (X² = 0.7, df = 1, NS; Figure 4a). The number of *L. salmonis* copepodids making directional responses, not choosing and the total number of replicates for each treatment are presented in Table 2d.

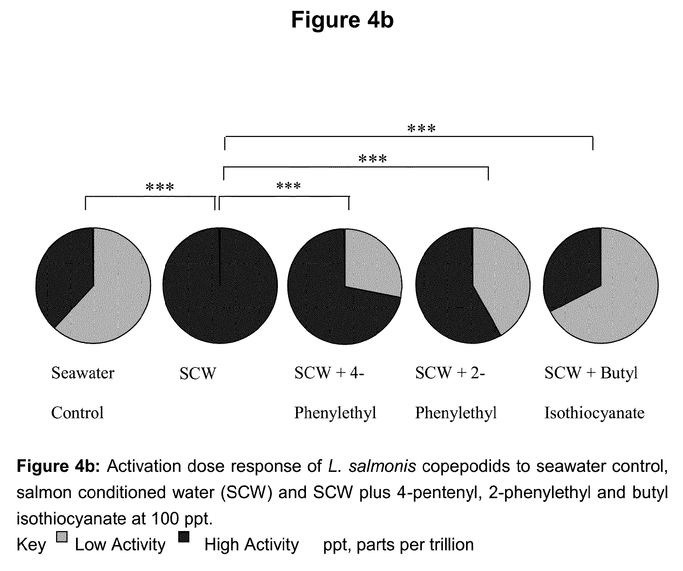

The global X² showed that lice did not behave the same in all activity treatments (X² = 97.56, df = 4, P < 0.001). When compared with the seawater control, a significant increase in high activity was detected with SCW (X² = 80.54, df = 1, P < 0.001). Significantly more copepodids showed low activity with SCW plus 100 ppt 4-pentenyl (X² = 25.97, df = 1, P < 0.001), 100 ppt 2-phenylethyl (X² = 41.40, df = 1, P < 0.001) and 100 ppt butyl isothiocyanate (X² = 75.42, df = 1, P < 0.001) when compared against SCW responses (Figure 4b).

**Table 2d**

| Number of *L. salmonis* copepodids making directional responses, non-choosers and the total number of replicates for each treatment. | | | |
|---|---|---|---|
| Assay | Directional Responses | No Choice | Total No. Replicates |
| ASW Control | 36 | 84 | 120 |
| ASW v SCW | 81 | 2 | 83 |
| ASW v SCW + 4-Pentenyl Isothiocyanate | 24 | 26 | 50 |
| ASW v SCW + 2-Phenylethyl Isothiocyanate | 15 | 35 | 50 |
| ASW v SCW + Butyl Isothiocyanate | 12 | 40 | 52 |

### Discussion:

In this study, it has been shown that copepodid larvae of the salmon louse, *L*. *salmonis,* show significant directional responses to isophorone, a component of salmon conditioned water. Isophorone has been identified as a behaviourally active component of salmon-conditioned water (Bailey *et al*., 2006) and was therefore used as a host cue to elicit a response in preliminary experiments. The inclusion of garlic oil with isophorone, removed the attraction to isophorone. On its own, garlic oil appears to repel lice to the artificial seawater. It is suggested here that garlic oil may act as a lice repellent and/or mask host odour cues, i.e. as a semiochemical masking and attraction reducing material.

We have also found that the addition of 50 and 10 parts per trillion diallyl sulphide removed the attraction to salmon conditioned water. Diallyl sulphide at 10 parts per trillion however appeared to be the more effective masking compound.

Further, we have shown that 2-phenylethyl, butyl and propyl isothiocyanate at the 100 parts per trillion concentration, removed the attraction of copepodids to salmon conditioned water. 4-pentenyl isothiocyanate did not mask copepodid responses to salmon conditioned water however. Dose response experiments with butyl isothiocyanate showed 100 parts per trillion to be the most effective concentration for switching off responses to salmon conditioned water. Allyl isothiocyanate dose response assays suggest a possible effect at 100 parts per trillion.

A high number of non-choosers were seen in all seawater controls and is due to a lack of cues to stimulate a behavioural response from the lice.

In general, the seawater controls showed predominantly low activity behaviour in copepodids. This switched to high activity in the presence of a positive cue i.e. either isophorone or salmon conditioned water. Low activity re-appeared in the profile when test compounds were introduced, suggesting that the chemicals masked the effect of the isophorone or salmon conditioned water in copepodids. The extent of masking was variable between compounds and is thought to be related to the original field source of *L. salmonis.*

### Conclusions from example 1:

The use of plant derived masking compounds has been shown to significantly disrupt *L. salmonis* copepodid attraction to host (salmon) conditioned water *in vitro.* By masking the profile of the key host recognition molecules it was surprisingly possible to significantly reduce the host response of both *L. salmonis* and C. *rogercresseyi.* In the shown series of Y-tube assessments, sea lice showed a significant activity towards host odours from control Atlantic salmon. Inclusion of a series of masking compounds of vegetable origin effectively reduced this response in both species. Diallyl sulphide, diallyl disulphide, butyl isothiocyanate, allyl isothiocyanate, propyl Isothiocyanate, rosemary oil, lavender oil and bog myrtle were identified as candidate compounds for masking salmon host compounds.

The following compounds and concentrations were especially promising: Diallyl sulphide (10parts per trillion) and diallyl disulphide (100parts per trillion).

### Example 2

Evaluation of the effect of masking compounds on chemicals cues released by Atlantic salmon

### Material and methods

### Fish conditioned water

Atlantic salmon, S. *salar* were hatchery-reared stock produced at the west coast of Puerto Montt (Chile). For the preparation of Salmon Conditioned Water (SCW), one fish-host (100-200 g) was placed in a flume during 24 h with artificial seawater (100 L) (Aquarium salt; SERA, Heinsberg/Germany) with a salinity of 32‰ at 12 °C. The flow rate in the flume was 30 cm s-1 (Ingvarsdottir et al., 2002b). The water kept in the flume was used for bioassays, or frozen for use in chemical analysis.

### Lice

Ovigerous C. rogercresseyi females were collected from freshly harvested Atlantic salmon, on commercial fish farms on the west coast of Puerto Montt (Chile). Egg strings were removed gently from their point of attachment to adult females using ultra-fine forceps and were placed in a 500 mL glass culture flask with artificial seawater and held in suspension by an air supply through the stem at 12 °C keep them in absolute darkness until the copepodid stage was reached.

### Semiochemical Masking Compounds

Butyl isothiocyanate (B1), Diallyl sulphide (B2) and Diallyl disulfide (B3) were selected as test compounds. Each of the compounds were prepared in three solution with ethanol 1.0; 0.01 and 0.001 mg mL⁻¹, then where diluted to 1 µL L-1 in Salmon Conditioned Water (SCW).

### Preference Bioassays

A vertical Y-tube bioassay modified from that previously described was used to study C. *rogercresseyi* copepodid activation and directional (taxis) responses to host odours. The Y tube was made from perspex. The arms were 5 cm in length and the main leg was 6 cm long.

Water flowed through into each arm from reservoirs positioned immediately above the Y tube at a rate of 2 mL·min-1. In control assays, artificial seawater was introduced into both arms of the Y tube. When salmon-conditioned water (SCW) plus masking compounds were tested, the test water was introduced into one arm while seawater was introduced into the other.

At the beginning of each experiment, the Y tube was allowed to fill, and a single copepodid was introduced by polytetrafluoroethylene tubing (1 mm internal diameter (i.d.)) and syringe into the tube at a point 1 cm above the base of the main arm.

The copepodid was allowed a maximum of 5 min to respond. Behaviour was defined by the degree of movement within the Y tube, as described previously. Behaviour was divided into two categories, low and high. Low activity was defined as the movement of the copepodid less than the length of the main leg. High activity was defined as movement of the copepodid more than the length of the main leg. Preference was observed when the copepodid with high activity choose either arm. Both activation and directional responses of copepodids were measured. Each trial consisted of one copepodid, and each copepodid was never used more than once. There were 30-100 trials conducted for each experiment.

### Results

**Table 1**

| Response of copepodids Caligus rogercresseyi to different concentration of masking compounds in a vertical Y-tube bioassays. | | | | | |
|---|---|---|---|---|---|
| | Activity (%) | | | | |
| | High | Low | X² | P | N |
| Control | 67.50 | 32.50 | 4.9 | 0.027 | 40 |
| SCW | 62.64 | 37.36 | 5.8 | 0.016 | 100 |
| B1. Butyl isotiocianate | | | | | |
| SCW + (0,001 mg/mL) | 68.63 | 31.37 | 7.1 | 0.008 | 50 |
| SCW + (0,01 mg/mL) | 62.86 | 37.14 | 4.2 | 0.031 | 70 |
| SCW + (1,00 mg/mL) | 69.00 | 31.00 | 4.7 | 0.029 | 70 |
| B2. Dialyl sulfide | | | | | |
| SCW + (0,001 mg/mL) | 86.67 | 13.33 | 24.2 | 0.000 | 45 |
| SCW + (0,01 mg/mL) | 85.00 | 15.00 | 19.6 | 0.000 | 40 |
| SCW + (1,00 mg/mL) | 93.33 | 6.67 | 22.5 | 0.000 | 30 |
| B3. Dialyl disulfide | | | | | |
| SCW + (0,001 mg/mL) | 80.65 | 19.35 | 24.2 | 0.000 | 31 |
| SCW + (0,01 mg/mL) | 67.50 | 32.50 | 4.9 | 0.027 | 40 |
| SCW + (1,00 mg/mL) | 93.33 | 6.67 | 22.5 | 0.000 | 30 |
| P > 0,05 (test X²). | | | | | |

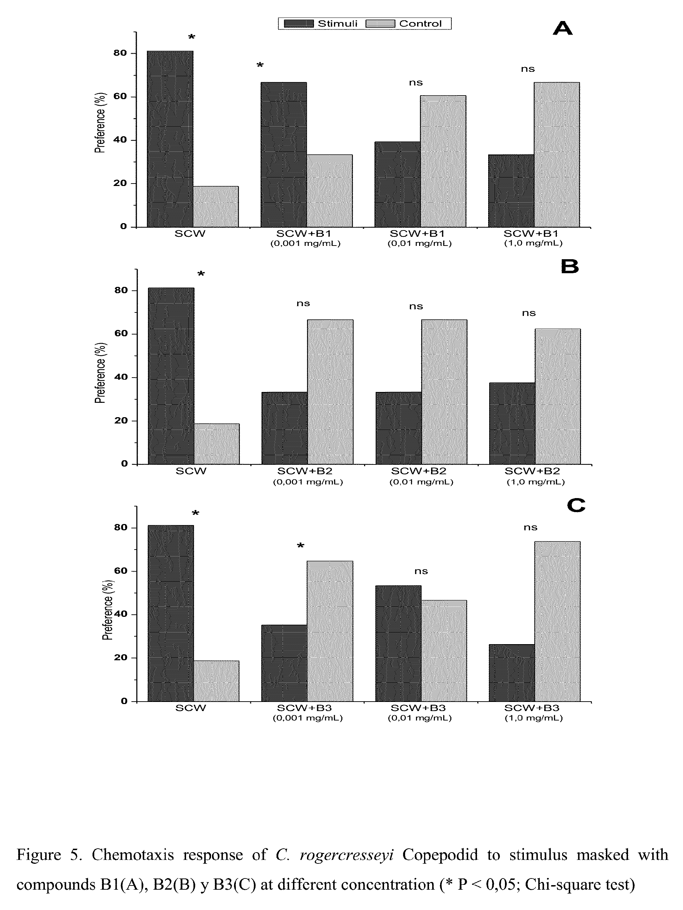

The level of preference was affected when the masking compounds were added. B1 at 0.01 and 1 mg / mL tends to change the preference shown by the sea lice at a lower concentration and control (Fig. 5A).

B2, at all the concentrations, showed a masking effect on the chemical cues released by Atlantic salmon, although no significant differences (Fig. 5B). B3 at 0.001 mg / mL significantly (P > 0.05) changed the preference of the copepodids (Fig. 5C).

An index of preference (IP) were calculated. IP = # visits at stimulus zones / # visits in the control zone. Which indicates, if IP = 1, there is no avoidance neither preference, if IP > 1, it indicates that there is preference for the stimulus, and if IP <1, it indicates an avoidance for the stimulus or a preference for control.

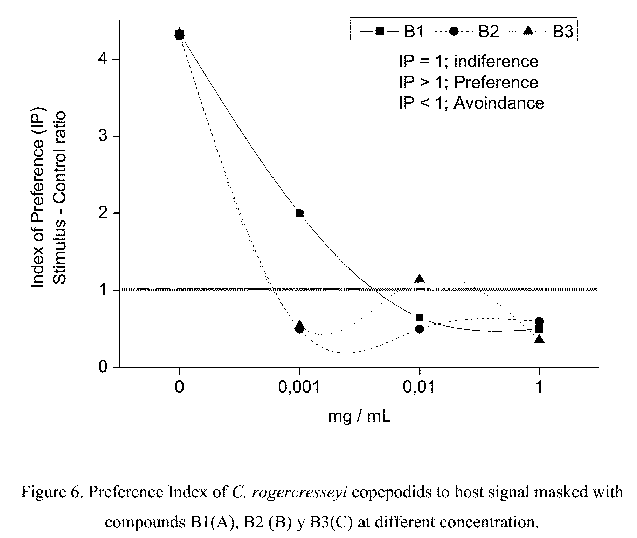

This study found that the IP calculated for B1 showed that the highest concentrations (0.01 and 1.0 mg mL-1) reduced the preference for the stimulus of Atlantic salmon. In the case of masked B2 and B3, the IP showed that both compounds were effective in their action of masking chemical cues (Fig. 6).

### Example 3

Effects of B's in feed on disruption of copepodids settlement of *Caligus rogercresseyi*

The aim of this experiment was to validate the effect of three masking compounds in feeds on the disruption of copepodid settlement and *in vivo* challenge assays.

### Materials and Method

### Semiochemical Masking Compounds

Isobutyl thiocyanate (B1), Diallyl sulfide (B2) and Diallyl disulfide (B3) were selected as test compounds.

### Tank Trails

### Fish

Atlantic salmon, *Salmo salar* (N = 168; 500g avg), hatchery-reared stock produced and maintained in Chile prior to the experiment, were in Chile, smolted gradually through a freshwater to seawater gradient and held in a circular tank (12 m³). Fish were pit-tagged at the end of smoltification.

### Sealice

100 ovigerous females *C. rogercresseyi* (5000 copepodids) for each tank were collected from freshly harvested Atlantic salmon, S. *salar* were placed in 2000 mL glass culture flasks with clean seawater, and held in suspension by an air supply through the stem at 12°C in absolute darkness. Egg strings were removed gently from their point of attachment using ultra-fine forceps and were placed in a 2000 mL glass culture flask with clean seawater and held in suspension with air supply at 12°C keep them in absolute darkness until the copepodid stage was reached. The emerged copepodid were used for infestation during the trail.

### Tank distribution.

14 fish, individually weight and tagged, were distributed in 12 fibreglass tanks (350L) with a flow through seawater (32 ‰) system at 13-14°C. Three tanks (replicas) were used for each masking compound dose and control diet.

### Masking compound feed formulation.

A dose of masking compounds (B1, B2 and B3) (0.125%) were tested against sea lice settlement compare with a commercial diet used as a Control. Feeding periods were held for 21 days, before sea lice infestation (Table 1). Post-Infestation feeding was held for 8 days.

**Table 1**

| Setting up experiments. | | | | |
|---|---|---|---|---|
| Feeding Tanks | Formulation | Number of Fish | Fish weight (g) | Feeding days) |
| 1,2,3 | FormB1 (0.125%) | 14 | 500 | 21 |
| 4,5,6 | FormB2 (0.125%) | 14 | 500 | 21 |
| 7, 8, 9 | FormB3 (0.125%) | 14 | 500 | 21 |
| 10, 11, 12 | Control | 14 | 500 | 21 |

### Sea lice Counting

Fish were culled and removed for the sampling. Sea lice were counted individually on each fish at 8 days post challenge.

### Results

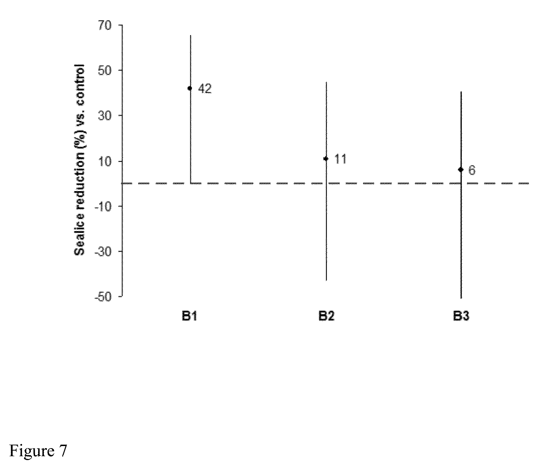

Fish fed the butyl isothiocyanate (B1) showed a significant reduction of 42% in levels of sea lice compared to controls (Figure 7). There was a trend for a reduction in lice levels with both diallyl sulfide (B2) and diallyl disulfide (B3)

It will be appreciated that the features of the invention described in the foregoing can be modified without departing from the scope of the invention.

### Definitions of terms:

The term "semiochemical" (semeon means a signal in Greek) is a generic term used for a chemical substance or mixture that carries a message. These chemicals acts as messengers for members of the same species or in some cases other species. It is usually used in the field of chemical ecology to encompass pheromones, allomones, kairomones, attractants and repellents. Please note especially that the term in respect of this application is not restricted to messengers between the same species, and that the term specifically is used to denote messengers between different species, such as between a Salmonidae and a parasite. The term is intended to include the chemical compounds which are specific for the attraction of parasites to Salmonidae, and especially to the attraction of sea lice to Salmonidae.

## Claims

1. An extract or compound for use in masking the odor of a fish semiochemical in water, wherein the attraction between a parasite and a fish in water is reduced,
**characterized in that** said extract or compound is added to said water or is administered to a fish in said water, wherein said extract or compound is selected from;
i) an extract of garlic, or
ii) a compound of formula (I);
**R¹-S-S-R¹** (I)
wherein each R¹ independently of each other is C₁-C₄ alkyl or C₂-C₃ alkenyl or C₂-C₃ alkynyl.

2. A composition according to claim 1, wherein said fish semiochemical is isophorone.

3. A composition according to claim 1, wherein said fish semiochemical is 1-Octen-3-ol or 6-methyl-5-hepten-2-one.

4. A composition according to claim 1, wherein said fish is a Salmonidae, preferably selected from the group consisting of Atlantic salmon, Coho salmon, Chinook, rainbow trout, Arctic char and other farmed salmon species.

5. A composition according to claim 1, wherein said parasite is an ectoparasite, and preferably sea lice (*Lepeophtheirus salmonis, Caligus* sp.*)*.

6. A compound for use in masking the odor of a fish semiochemical in water, wherein the attraction between a parasite and a fish is r reduced or wherein the infestation or infection of a parasite in a fish is reduced, or **characterized in that** said compound is added to said water or is administered to a fish in said water, wherein said compound is a compound of formula (I);
**R¹-S-S-R¹** (I)
wherein each R¹ independently of each other is C₁-C₄ alkyl or C₂-C₃ alkenyl or C₂-C₃ alkynyl, and wherein said parasite is an ectoparasite, preferably sea lice *(Lepeophtheirus salmonis, Caligus* sp.*)*.

7. A composition according to claim 6, wherein said fish is a Salmonidae, preferably selected from the group consisting of Atlantic salmon, Coho salmon, Chinook, rainbow trout, Arctic char and other farmed salmon species.

8. A feed composition for use in masking the odor of a fish semiochemical in water, wherein the attraction between a parasite and a fish is reduced, or wherein the infestation or infection of a parasite in a fish is reduced, wherein the feed comprises conventional feed ingredients such as lipids, proteins, vitamins, carbohydrates and minerals, and a compound of formula (I);
**R¹-S-S-R¹** (I)
wherein each R¹ independently of each other is C₁-C₄ alkyl or C₂-C₃ alkenyl or C₂-C₃ alkynyl.

9. A feed composition according to claim 8, said compound or extract in the feed are in a concentration range of 0.01-0,5, preferably in a concentration of 0.125% by weight of the feed.

## Patentansprüche

1. Extrakt oder Verbindung zur Verwendung bei der Überdeckung des Geruches einer Fisch-Semiochemikalie in Wasser, wobei die Anziehung zwischen einem Parasiten und einem Fisch im Wasser verringert wird, **dadurch gekennzeichnet, dass** der Extrakt oder die Verbindung zu dem Wasser hinzugefügt werden oder einem Fisch in dem Wasser verabreicht werden, wobei der Extrakt oder die Verbindung aus Folgendem ausgewählt ist:
i) Knoblauchextrakt, oder
ii) Verbindung der Formel (I):
**R¹-S-S-R¹** (I)
wobei es sich bei jedem R¹ unabhängig voneinander um C₁-C₄-Alkyl oder C₂-C₃-Alkenyl oder C₂-C₃-Alkynyl handelt.

2. Zusammensetzung gemäß Anspruch 1, wobei es sich bei der Fisch-Semiochemikalie um Isophoron handelt.

3. Zusammensetzung gemäß Anspruch 1, wobei es sich bei der Fisch-Semiochemikalie um 1-Octen-3-ol oder 6-Methyl-5-hepten-2-on handelt.

4. Zusammensetzung gemäß Anspruch 1, wobei es sich bei dem Fisch um Salmonidae handelt, die bevorzugt ausgewählt sind aus der Gruppe bestehend aus Atlantischem Lachs, Silberlachs, Königslachs, Regenbogenforelle, Seesaibling und anderen gezüchteten Lachsarten.

5. Zusammensetzung gemäß Anspruch 1, wobei es sich bei dem Parasiten um einen Ektoparasiten, und bevorzugt Meerläuse (*Lepeophtheirus salmonis*, *Caligus* sp.) handelt.

6. Verbindung zur Verwendung bei der Überdeckung des Geruches einer Fisch-Semiochemikalie in Wasser, wobei die Anziehung zwischen einem Parasiten und einem Fisch verringert wird oder wobei der Befall oder die Infektion eines Fisches mit einem Parasiten verringert wird, oder **dadurch gekennzeichnet, dass** die Verbindung zu dem Wasser hinzugefügt wird oder einem Fisch in dem Wasser verabreicht wird, wobei es sich bei der Verbindung um eine Verbindung der Formel (I) handelt;
**R¹-S-S-R¹** (I)
wobei es sich bei jedem R¹ unabhängig voneinander um C₁-C₄-Alkyl oder C₂-C₃-Alkenyl oder C₂-C₃-Alkynyl handelt, und wobei es sich bei dem Parasiten um einen Ektoparasiten, bevorzugt Meerläuse (*Lepeophtheirus salmonis, Caligus* sp.) handelt.

7. Zusammensetzung gemäß Anspruch 6, wobei es sich bei dem Fisch um Salmonidae handelt, die bevorzugt ausgewählt sind aus der Gruppe bestehend aus Atlantischem Lachs, Silberlachs, Königslachs, Regenbogenforelle, Seesaibling und anderen gezüchteten Lachsarten.

8. Futterzusammensetzung zur Verwendung bei der Überdeckung des Geruchs einer Fisch-Semiochemikalie in Wasser, wobei die Anziehung zwischen einem Parasiten und einem Fisch verringert wird oder wobei der Befall oder die Infektion eines Fisches mit einem Parasiten verringert wird, wobei das Futter herkömmliche Futterzutaten, wie Fette, Proteine, Vitamine, Kohlenhydrate und Mineralien sowie eine Verbindung der Formel (I) umfasst;
**R¹-S-S-R¹** (I)
wobei es sich bei jedem R¹ unabhängig von einander um C₁-C₄ -Alkyl oder C₂-C₃-Alkenyl oder C₂-C₃-Alkynyl handelt.

9. Futterzusammensetzung gemäß Anspruch 8, wobei die Verbindung oder der Extrakt im Futter in einem Konzentrationsbereich von 0,01 - 0,5, vorzugsweise in einer Konzentration von 0,125 Gewichtsprozent des Futters vorliegen.

## Revendications

1. Extrait ou composé servant à masquer fodeur d'un poisson sémiochimique dans l'eau, où l'attraction entre un parasite et un poisson dans l'eau est réduite, **caractérisé en ce que** ledit extrait ou composé est ajouté à ladite eau ou est administré à un poisson dans ladite eau, où ledit extrait ou composé est choisi parmi ;
i) un extrait d'ail, ou
ii) un composé de formule (I) ;
**R¹-S-S-R¹** (I)
où chaque R¹ indépendamment l'un de l'autre est alkyle C₁-C₄ ou alkényle C₂-C₃ ou alkynyle C₂-C₃.

2. Composé selon la revendication 1, où ledit poisson sémiochimique est isophorone.

3. Composition selon la revendication 1, où ledit poisson sémiochimique est 1-octèn-3-ole ou de 6-méthyle-5-heptèn-2-one.

4. Composition selon la revendication 1, où ledit poisson est un salmonidé, de préférence choisi parmi le groupe comprenant le saumon de l'Atlantique, le saumon coho, le saumon quinnat, la truite arc-en-ciel, l'omble de l'Arctique et d'autres espèces de saumon d'élevage.

5. Composition selon la revendication 1, où ledit parasite est un ectoparasite, et de préférence le pou du poisson (*Lepeophtheirus salmonis, Caligus* sp.).

6. Composé servant à masquer l'odeur d'un poisson sémiochimique dans l'eau, où l'attraction entre un parasite ou un poisson est réduite ou où l'infestation ou l'infection d'un parasite dans un poisson est réduite, ou **caractérisé en ce que** ledit composé est ajouté à ladite eau ou est administré à un poisson dans ladite eau, où ledit composé est un composé de formule (I) ;
**R¹-S-S-R¹** (I)
où chaque R¹ indépendamment l'un de l'autre est alkyle C₁-C₄ ou alkényle C₂-C₃ ou alkynyle C₂-C₃ et où ledit parasite est un ectoparasite, et de préférence le pou du poisson (*Lepeophtheirus salmonis, Caligus* sp.).

7. Composition selon la revendication 6, où ledit poisson est un salmonidé, de préférence choisi parmi le groupe comprenant le saumon de l'Atlantique, le saumon coho, le saumon quinnat, la truite arc-en-ciel, l'omble de l'Arctique et d'autres espèces de saumon d'élevage.

8. Composition alimentaire servant à masquer l'odeur d'un poisson sémiochimique dans l'eau, où l'attraction entre un parasite et un poisson est réduite, où l'infestation ou l'infection d'un parasite dans un poisson est réduite, où l'alimentation comprend des ingrédients alimentaires conventionnels comme les lipides, les protéines, les vitamines, les carbohydrates et les minéraux, et un composé de formule (I) ;
**R¹-S-S-R¹** (I)
où chaque R¹ indépendamment l'un de l'autre est alkyle C₁-C₄ ou alkényle C₂-C₃ ou alkynyle C₂-C₃.

9. Composition alimentaire selon la revendication 8, ledit composé ou ledit extrait dans l'alimentation ont une plage de concentration de 0,01-0,5, de préférence une concentration de 0,125 % en poids de l'aliment.
